# FASCICULE DE BREVET EUROPEEN

(11) **EP 1 374 763 B1**
(45) Date de publication et mention de la délivrance du brevet: **26.09.2007**
(21) Numéro de dépôt: 03291464.0
(22) Date de dépôt: 17.06.2003
(51) Int. Cl.: A61B 5/024

(54) **Equipement portable destiné à la mesure et/ou la surveillance de la fréquence cardiaque**
Tragbare Ausrüstung zur Messung und Überwachung der Herzfrequenz
Portable equipment for heart rate measuring and monitoring

(30) Priorité: 18.06.2002 FR 0207465
(43) Date de publication de la demande: 02.01.2004
(73) Titulaire: CSEM Centre Suisse d'Electronique et de Microtechnique S.A. - Recherche et Développement, 2007 Neuchâtel (CH)
(72) Inventeur: Verjus, Christophe, 2000 Neuchatel (CH); Vetter, Rolf, 1025 Monnaz (CH); Celka, Patrick, 1072 Forel (CH); Renevey, Philippe, 1005 Lausanne (CH)
(74) Mandataire: Colas, Jean-Pierre

(56) Documents cités:
- EP-A- 0 923 903
- EP-A- 0 941 694
- WO-A-97/14357

## Description

La présente invention est relative à un équipement portable de mesure optique et/ou de surveillance de la fréquence cardiaque.

Il existe actuellement de nombreuses variantes d'équipements de ce type. Ils comportent une sonde optique destinée à être placée sur une région de la peau d'un utilisateur. Cette sonde comprend un émetteur rayonnant un flux lumineux dans le tissu de cette région et un récepteur capable de capter l'énergie provenant du tissu (par exemple par réflexion sur ou par transmission à travers le tissu) dont les caractéristiques optiques varient en fonction de la circulation sanguine. Le signal fourni par le récepteur est analysé et débarrassé des artefacts dus notamment aux mouvements de l'utilisateur, à la lumière ambiante, etc., dans une unité de traitement électronique dont le signal de sortie peut être rendu perceptible de toute manière appropriée, par exemple par affichage sur un écran, par restitution sonore ou autre. Le signal de sortie peut être confronté à un seuil dont le franchissement par la fréquence cardiaque peut déclencher une alarme.

Un équipement présentant ces caractéristiques est décrit notamment dans EPA-1 297 784 (état de la technique selon l'article 54(3) CBE). Ce document décrit en particulier une sonde destinée à être portée au poignet, le signal utile étant extrait grâce à l'utilisation d'un dispositif de détection des mouvements du porteur.

Dans une autre réalisation connue, la sonde est incorporée dans une ceinture pectorale et le signal utile est transmis par radio à un dispositif d'affichage, tel celui d'une montre-poignet.

Par les documents WO 97/14357 ou EP 0 923 903 A1 sont connus des dispositifs conçus pour être fixés sur l'oreille et dont la sonde est destinée à être portée au contact du lobe de l'oreille.

L'invention a pour but de fournir un équipement du genre indiqué ci-dessus qui fournisse une information fiable concernant la fréquence cardiaque de son porteur, tout en étant commode d'utilisation et particulièrement adapté pour être utilisé par des personnes pendant l'exercice d'une activité, comme les sportifs, par exemple.

L'invention a donc pour objet un équipement portable caractérisé en ce qu'il comprend en combinaison un dispositif de mesure de la fréquence cardiaque, et une unité de restitution sonore comprenant des moyens fournissant un signal représentatif de la restitution sonore pour fournir à un transducteur sonore de l'information sonore, ledit dispositif de mesure et ledit transducteur sonore étant montés au moins partiellement dans un ensemble conçu pour être fixé sur l'oreille d'un porteur de l'équipement, et ladite unité de restitution sonore comprenant des moyens pour substituer audit signal représentatif de la restitution sonore et/ou superposer sur ce signal, un signal engendré à partir des signaux dudit dispositif de mesure et représentatif de ladite fréquence cardiaque ledit ensemble conçu pour être fixé sur l'oreille d'un porteur se présentant sous la forme d'une oreillette, présentant un boîtier logeant ledit transducteur et destiné à être placé devant l'embouchure du conduit auditif d'un porteur et une corne solidaire du boîtier et destinée à être placée derrière le pavillon de l'oreille d'un porteur, ledit dispositif de mesure étant logé en partie dans ledit boîtier et en partie dans ladite corne, ledit ensemble comportant en outre un dispositif accélérométrique délivrant des signaux de mouvement représentatifs du mouvement du porteur lorsque ledit ensemble est fixé sur l'oreille dudit porteur et ledit dispositif accélérométrique comportant en outre des moyens de traitement de signal utilisant lesdits signaux de mouvement pour corriger des artéfacts dus au mouvement dans ledit signal représentatif de ladite fréquence cardiaque.

Grâce à ces caractéristiques, la fonction de mesure de la fréquence cardiaque peut être associée à celle d'un appareil de reproduction acoustique, comme un baladeur par exemple. De tels appareils de reproduction acoustique sont d'un usage très répandu, notamment par les sportifs exerçant leur activité. L'information concernant la fréquence cardiaque est ainsi fournie en tant que complément au signal de reproduction acoustique, en général d'agrément, que le porteur de l'équipement a l'habitude d'écouter. En outre, la présentation de cette information complémentaire se fait par l'intermédiaire d'une composante (l'oreillette) d'un équipement de reproduction acoustique qui est habituelle et qui se met en place sur l'oreille du porteur de l'équipement de la façon habituelle, la forme extérieure de la sonde placée sur l'oreille n'étant, à dessein, pas différente de celle d'une oreillette habituelle. En outre, la fonction de mesure de la fréquence cardiaque est mise en oeuvre de façon très discrète, ce qui peut être ressentie comme un avantage par les utilisateurs de l'équipement. On notera enfin qu'une oreillette se maintient en place sans problème malgré les mouvements du porteur, tandis que la connectique entre l'oreillette et le boîtier du baladeur n'est également, du moins extérieurement, pas modifiée et nécessite seulement un câble de connexion légèrement modifié par rapport aux câbles usuel. Il peut donc être très robuste.

Les équipements portables connus, outre le fait que certains sont sous forme de clip et pincent le lobe de l'oreille et donc empêchent sa vascularisation, comprennent un capteur destiné à être porté au contact d'une partie souple de l'oreille (le lobe) et il est donc difficile dans ces dispositifs d'éliminer les artéfacts liés aux mouvements du corps du porteur. Conformément à la présente invention, l'unité détectrice comprenant le capteur étant intégrée dans une oreillette, elle est donc avantageusement destinée à être portée au contact d'une partie rigide de l'oreille et il devient donc par là même plus facile d'éliminer les artéfacts créés par le mouvement du corps.

Selon d'autres caractéristiques avantageuses de l'invention:
- ledit dispositif de mesure comprend un émetteur de rayonnement optique et un récepteur de rayonnement optique placés respectivement dans ledit boîtier et dans ladite corne, le trajet lumineux entre eux pouvant ainsi passer à travers une portion du pavillon de l'oreille d'un porteur;
- ledit émetteur de rayonnement optique comprend une pluralité de sources lumineuses émettant sur des longueurs d'onde distinctes, situées de préférence dans le proche infrarouge;
- ledit récepteur comprend autant de groupes de détecteurs de rayonnement optique qu'il y a de sources de lumière dans l'émetteur et ledit dispositif de mesure comprend également des moyens pour calculer la moyenne des signaux fournis par les détecteurs de chaque groupe;
- lesdits moyens de substitution/superposition de ladite unité de restitution sonore comprennent au moins un circuit de mixage connecté pour recevoir ledit signal représentatif de la fréquence cardiaque fourni par ledit dispositif de mesure, ledit circuit de mixage étant connecté entre ledit transducteur sonore et lesdits moyens fournissant ledit signal représentatif de la restitution sonore;
- ladite unité de restitution sonore comprend des moyens de synthèse vocale connectés entre ladite sonde et ledit circuit de mixage;
- l'équipement comprend un comparateur dont une première entrée reçoit le signal représentatif de la fréquence cardiaque et dont une seconde entrée est reliée à un générateur de seuil dont le seuil est représentatif d'une valeur prédéterminée de la fréquence cardiaque, et dont la sortie est reliée audit circuit de mixage pour envoyer dans ledit transducteur sonore une information d'alarme, lorsque ladite valeur prédéterminée est dépassée par la valeur de la fréquence cardiaque fournie par ledit dispositif de mesure;
- un sélecteur permet de choisir la reproduction dans ledit transducteur sonore soit de ladite information d'alarme, soit dudit signal représentatif de la fréquence cardiaque fournie par ledit dispositif de mesure;
- ladite unité de restitution sonore comprend un lecteur d'un support d'information sonore tel qu'une cassette audio, un CD ou un DVD, connecté audit ensemble par un câble ;
- ladite unité de restitution sonore comprend un microphone il constitue une prothèse acoustique.

D'autres caractéristiques et avantages de la présente invention apparaîtront au cours de la description qui va suivre, donnée uniquement à titre d'exemple et faite en se référant aux dessins annexés sur lesquels:
la figure 1 est une représentation schématique d'un équipement portable permettant une reproduction acoustique combinée à la mise en oeuvre d'une fonction de mesure et/ou de surveillance de la fréquence cardiaque;
la figure 2 est un schéma synoptique de l'équipement représenté sur la figure 1; et
la figure 3 représente schématiquement un autre mode de réalisation de l'invention.

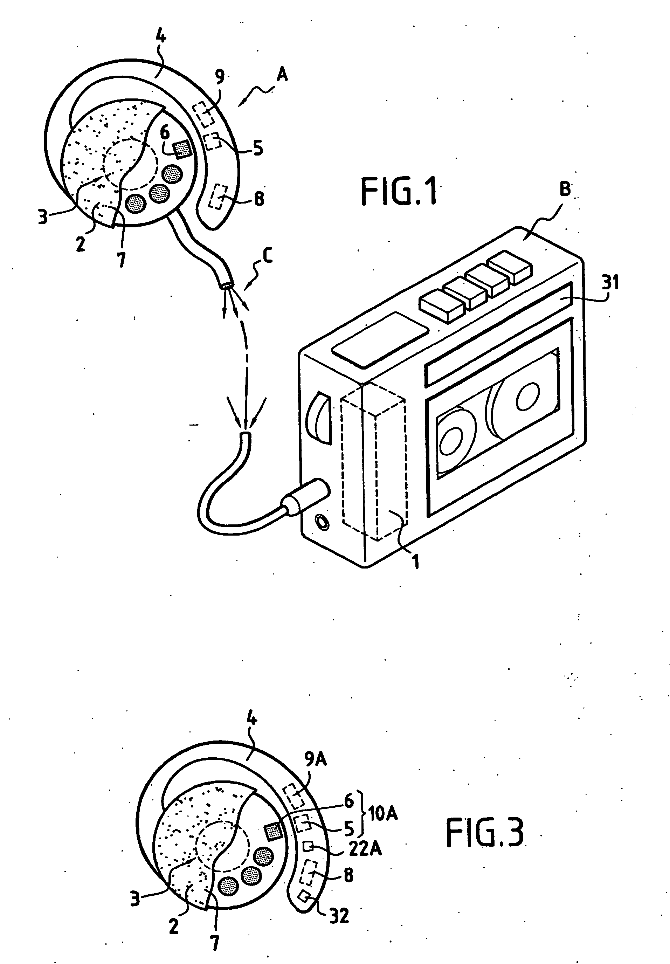

La figure 1 représente un premier mode de réalisation d'un équipement portable selon l'invention.

Cet équipement comprend une première unité A destinée notamment à mesurer la fréquence cardiaque et une seconde unité de restitution sonore B destinée notamment à assurer une reproduction acoustique, les deux unités étant connectées entre elles par un câble C. Dans le mode de réalisation représenté, l'unité B est un lecteur de cassettes audio, de préférence agencé pour être facilement porté par un utilisateur, comme cela est courant pour les appareils appelés couramment "baladeurs". Bien entendu, l'invention s'applique à d'autres appareils de reproduction acoustique, fonctionnant avec d'autres supports d'enregistrement connus tels que les CD, les DVD et autres. Le lecteur de cassettes B est de conception classique et n'est donc pas décrit en détail. Il suffit de noter simplement que l'on complète son circuit électrique de reproduction acoustique avec des éléments qui sont symboliquement représentés sur la figure 1 par un bloc fonctionnel 1 à décrire par la suite.

L'unité A se présente sous la forme d'une oreillette comprenant un boîtier 2 de forme générale circulaire et destiné à être placé dans le pavillon de l'oreille d'un utilisateur devant l'embouchure du conduit auditif, la face visible sur la figure 1 du boîtier 2 étant tournée vers cette embouchure. Le boîtier 2 de l'oreillette renferme également un transducteur sonore indiqué symboliquement par le cercle en pointillés 3. Ce transducteur est classique, tout modèle connu disponible sur le marché pouvant convenir.

Le boîtier 2 est solidaire d'une corne 4 dont la partie d'extrémité libre est destinée à venir s'accrocher derrière le pavillon de l'oreille d'un utilisateur, comme cela est courant pour les oreillettes utilisées avec les baladeurs du commerce. Un émetteur de rayonnement optique 5 est placé dans la corne 4 de manière telle que le rayonnement émis soit dirigé vers le boîtier 2. En d'autres termes, en considérant la figure 1, cet émetteur 5 est placé dans la face non visible de la corne 4.

Un récepteur de rayonnement optique 6 est disposé dans le boîtier 2 de telle manière qu'il puisse capter la composante du rayonnement optique provenant de l'émetteur 5 et ayant traversé l'épaisseur du pavillon de l'oreille. Par conséquent, la composante de rayonnement reçu est fonction des variations des caractéristiques optiques de la portion du pavillon à travers laquelle passe ce rayonnement, variations qui sont dues notamment à celles de la circulation sanguine dans le pavillon. La variation du signal électrique délivré par le récepteur 6 est donc représentative notamment de la pulsation du sang et donc, de la fréquence cardiaque.

Le boîtier 2 peut être revêtu d'un tampon 7 en matière mousse (qui n'est représenté que partiellement) afin de protéger la partie de l'oreille entourant l'embouchure du conduit auditif externe.

Un dispositif accélérométrique 8 mesurant l'accélération selon trois axes est placé dans la corne 4. Son rôle sera commenté par la suite.

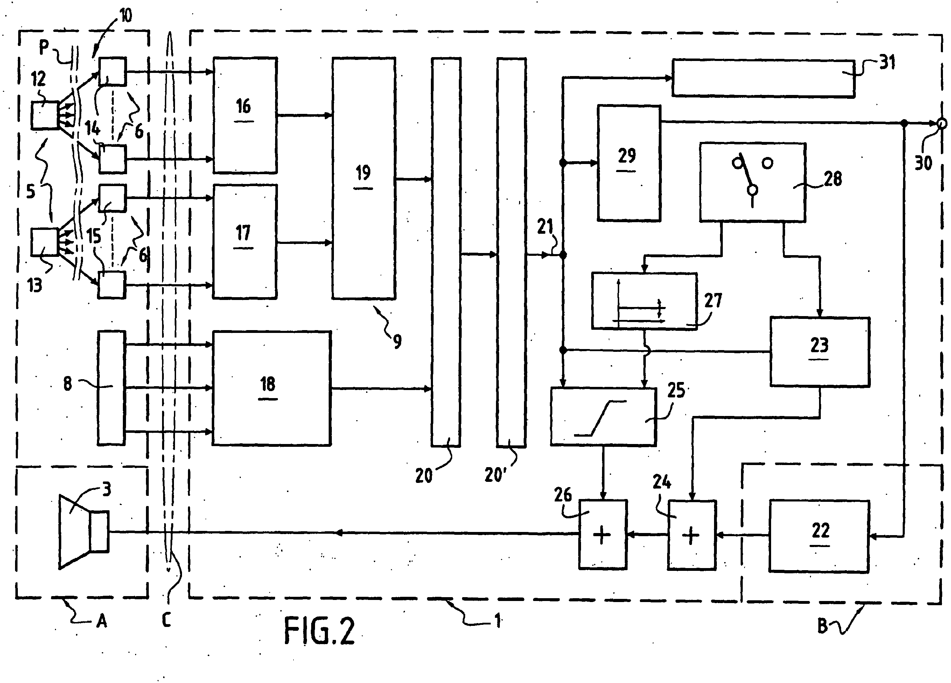

La figure 2 représente un schéma fonctionnel de l'équipement représenté sur la figure 1. On voit que l'unité A comprend le transducteur de restitution sonore 3 et une sonde de mesure 10. La sonde de mesure 10 comprend l'émetteur 5, le récepteur 6 et le dispositif accélérométrique 8. L'émetteur 5 comprend une ou plusieurs (deux dans le cas représenté) sources de rayonnement optique 12 et 13 émettant, par exemple, sur des longueurs d'onde différentes, en l'occurrence deux longueurs d'onde λ₁ et λ₂. Ces sources irradient une portion P du cartilage du pavillon de l'oreille. Comme déjà décrit, le récepteur 6 est situé du côté opposé de cette portion P du pavillon de l'oreille, lorsque l'oreillette est en place sur celle-ci.

En correspondance avec chaque source 12, 13, le récepteur 6 comprend une pluralité de détecteurs optiques, 14 et 15 respectivement, dont les gammes de sensibilité sont ajustées sur les longueurs d'onde respectives des sources 12 et 13. Selon une variante de réalisation, les sources 12 et 13 sont modulées par un signal de commande de manière à être actives alternativement. Des récepteurs 14 et 15 à large bande peuvent alors être utilisés et un démultiplexage temporel permettra de retrouver les deux signaux optiques de longueur d'onde λ₁ et λ₂.

Dans le mode de réalisation préféré de l'invention, les longueurs d'ondes λ₁ et λ₂ utilisées sont situées dans le proche infra-rouge, les sources étant constituées par des diodes électroluminescentes et les détecteurs par des diodes photosensibles.

Les sorties des groupes de détecteurs 14 et 15, du dispositif accélérométrique 8 et du transducteur 3 sont reliées par un câble C à une section de traitement désignée par la référence générale 9 et étant intégrée dans le bloc fonctionnel 1 incorporé dans l'unité B. Dans le contexte de la présente invention on désigne l'ensemble de la section de traitement 9 et de la sonde 10 par "dispositif de mesure de la fréquence cardiaque".

Les groupes de détecteurs 14 et 15 sont reliés respectivement à des moyens de filtrage et de conformation de signal 16 et 17 prévus dans la section de traitement 9. Le filtrage peut être réalisé par un filtre passe-bande analogique d'une bande passante de 0,5 à 10 Hz, par exemple. La conformation des signaux peut impliquer un moyennage sur les signaux analogiques fournis par chaque groupe de détecteurs 14 et 15, ainsi qu'une amplification adéquate des signaux.

Les signaux engendrés par le dispositif accélérométrique 8 sont appliqués à des moyens de filtrage et de conformation de signal 18 qui assurent un filtrage et une amplification de ces signaux, le filtrage étant réalisé à l'aide d'un filtre passe-bande analogique dont la bande passante peut être de 0,5 à 10 Hz, par exemple.

Les signaux ainsi traités issus des blocs 16 et 17 sont appliqués à un circuit d'élimination d'artefacts 19 dans lesquels ils subissent une conversion analogique/numérique, ainsi qu'un traitement d'élimination d'artefacts qui peut être celui décrit dans le document WO 99/32030. Ce traitement connu en soi consiste essentiellement à discriminer dans les signaux ceux dus aux battements cardiaques de ceux résultant des autres variations de l'écoulement sanguin.

Dans un bloc 20, les signaux issus des blocs 18 et 19 sont ensuite traités pour une analyse pendant laquelle, grâce aux signaux délivrés par le dispositif accélérométrique et prétraités par les moyens de filtrage et de conformation de signal 18, sont éliminés les artefacts dûs aux mouvements du porteur de l'équipement. Cette analyse est décrite en détail dans la demande de brevet européen précité notamment à propos de la figure 4 de ce document auquel on pourra se référer pour plus de détail.

Le signal issu du bloc d'analyse 20 est appliqué à un bloc 20', lequel effectue un calcul d'estimation de la fréquence cardiaque en déterminant, par exemple, la fréquence des pics d'intensité du signal issu du bloc 20. Le signal de sortie du bloc 20', apparaissant sur une borne 21, est ainsi représentatif du battement ou de la fréquence cardiaque, signal qui est débarrassé de tous les artefacts pouvant influencer le résultat de la mesure. Comme indiqué précédemment, l'unité B de restitution sonore est connectée au transducteur 3 par l'intermédiaire du câble C. Dans ce dernier, il est prévu également des conducteurs d'alimentation de l'unité A à partir de la source d'énergie (non représentée) se trouvant classiquement dans l'unité de restitution sonore B.

Il est à noter que cette unité B peut être agencée pour reproduire en stéréo les informations sonores qu'elle est destinée à reproduire à partir du support d'enregistrement que l'utilisateur y aura placé. Par conséquent, il peut être prévu un deuxième transducteur sonore se trouvant dans une deuxième oreillette destinée à l'autre oreille du porteur, comme cela est classique dans le cas des baladeurs disponibles dans le commerce. Par souci de simplification, ces éléments n'ont été ni représentés, ni décrits.

Sur la figure 2, le bloc 22 symbolise l'ensemble des moyens permettant de lire un support d'enregistrement et de fournir un signal représentatif d'une restitution sonore de ce qui est enregistré sur le support d'enregistrement; ces moyens étant classiques, ils ne sont pas décrits en détail.

Selon l'invention, le signal apparaissant sur la borne 21 et représentatif du battement cardiaque détecté est superposable sur les signaux sonores provenant de l'enregistrement que l'utilisateur souhaite écouter. A cet effet, le bloc fonctionnel 1 déjà représenté sur la figure 1 assure également diverses autres fonctionnalités que celles déjà décrites, ces autres fonctionnalités étant mises en oeuvre dans les blocs représentés à droite sur la figure 2. Ces fonctionnalités sont décrites à titre d'exemple pour illustrer le mode de réalisation préféré de l'invention. Cependant celle-ci ne se limite pas à cette description, l'invention pouvant impliquer soit des versions simplifiées, soit des versions plus complexes réalisant des fonctionnalités complémentaires.

Ceci étant, dans la version représentée, la borne 21 est connectée d'abord à un synthétiseur vocal 23. Ce dernier est relié à un premier circuit de mixage 24 placé sur le trajet de communication entre le bloc 22 et le transducteur 3. Ainsi le signal de fréquence cardiaque peut être superposé aux signaux sonores produits par le bloc de reproduction acoustique 22, par exemple avec une périodicité donnée que l'on peut éventuellement rendre réglable.

La borne 21 est également raccordée à une première entrée d'un comparateur 25 dont la sortie est reliée à un deuxième circuit de mixage 26 placé également sur le trajet par lequel transitent les signaux sonores vers le transducteur 3. La deuxième entrée du comparateur 25 est connectée à un générateur de seuil 27 destiné à déterminer un seuil d'alarme correspondant à une valeur de fréquence cardiaque jugée dangereuse pour le porteur de l'équipement. Le générateur de seuil 27 permet de préférence un réglage de ce seuil d'alarme. Par conséquent, lorsque la fréquence cardiaque mesurée dépasse le seuil d'alarme, un signal d'alarme peut être superposé aux signaux sonores transmis vers le transducteur 3.

De préférence, les fonctions de synthèse vocale et de comparaison à un seuil d'alarme que l'on vient de décrire pourront être choisies à volonté par le porteur de l'équipement. A cet effet, il est prévu un sélecteur 28 accessible par exemple par une touche (non représentée) prévue sur le boîtier de l'unité B.

La borne 21 peut également être connectée à un bloc fonctionnel 29 permettant d'enregistrer l'évolution de la fréquence cardiaque afin de garder trace de celle-ci. Avantageusement, cet enregistrement pourra être fait sur le support d'enregistrement qui est en cours de lecture dans le bloc 22 de l'unité B. Le bloc fonctionnel 29 peut être connecté à une borne de sortie 30 de l'unité B par l'intermédiaire de laquelle celle-ci peut être connectée à un dispositif de traitement d'information extérieur (non représenté) destiné par exemple à une analyse médicale de l'enregistrement de la fréquence cardiaque.

Enfin, il est prévu avantageusement un écran d'affichage 31 (voir également la figure 1) destiné à rendre visible le signal apparaissant sur la borne 21.

La figure 3 représente un autre mode de réalisation de l'invention dans lequel l'équipement constitue une prothèse auditive. Dans ce cas, il est prévu une unité de restitution sonore 22A comprenant un microphone 32 captant le son ambiant. Cette unité de restitution sonore 22A amplifie le signal du microphone et envoie le signal en résultant au transducteur 3 pour le porter à un niveau suffisant pour qu'un porteur malentendant puisse le percevoir. Cette transmission se fait par l'intermédiaire d'au moins un circuit de mixage (non représenté sur la figure 3) mais analogue aux circuits de mixage 24 et 26 représentés sur la figure 2.

Comme dans le mode de réalisation des figures 1 et 2, la sonde de mesure 10A comprend un émetteur 5 et un récepteur 6 pouvant être conçus conformément à ce qui a déjà été décrit ci-dessus à propos de la figure 2. Elle coopère avec une section de traitement 9A qui peut être analogue à la section de traitement 9 décrite ci-dessus.

Comme le montre la figure 3, la prothèse auditive est agencée dans une oreillette dont la structure est celle de l'oreillette déjà décrite. Cependant, dans ce cas, l'ensemble des composants de l'équipement et notamment la section de traitement 9A et l'essentiel des autres composants tels que les blocs 23 et 24 de la figure 2, est réparti dans le boîtier 2 et la corne 4 en étant alimenté par une pile (non représentée) comme cela est classique dans la technique des prothèses auditives.

## Revendications

1. Equipement portable comprenant en combinaison :
- un dispositif de mesure de la fréquence cardiaque (9, 10; 9A, 10A),
- une unité de restitution sonore (B; 22A, 31) comprenant des moyens fournissant un signal représentatif de la restitution sonore (22; 22A 31) pour fournir à un transducteur sonore (3) de l'information sonore,
- ledit dispositif de mesure (9, 10; 9A, 10A) et ledit transducteur sonore (3) étant montés au moins partiellement dans un ensemble (2, 4) conçu pour être fixé sur l'oreille d'un porteur de l'équipement, et
- ladite unité de restitution sonore (B; 22A, 31) comprenant des moyens (24, 26) pour substituer audit signal représentatif de la restitution sonore et/ou superposer sur ce signal, un signal engendré à partir des signaux dudit dispositif de mesure (9, 10, 9A, 10A) et représentatif de ladite fréquence cardiaque,
- ledit ensemble (2, 4) conçu pour être fixé sur l'oreille d'un porteur se présentant sous la forme d'une oreillette, présentant un boîtier (2) logeant ledit transducteur (3) et destiné à être placé devant l'embouchure du conduit auditif d'un porteur et une corne (4) solidaire du boîtier (2) et destinée à être placée derrière le pavillon de l'oreille d'un porteur,
**caractérisé en ce que :**
- ledit dispositif de mesure (9, 10; 9A, 10A) est logé en partie dans ledit boîtier (2) et en partie dans ladite corne (4),
- ledit ensemble comporte en outre un dispositif accélérométrique (8) délivrant des signaux de mouvement représentatifs du mouvement du porteur lorsque ledit ensemble est fixé sur l'oreille dudit porteur et ledit dispositif accélérométrique comporte en outre des moyens de traitement de signal (18, 20) utilisant lesdits signaux de mouvement pour corriger des artéfacts dus au mouvement dans ledit signal représentatif de ladite fréquence cardiaque.

2. Equipement portable suivant la revendication 1, **caractérisé en ce que** ledit dispositif de mesure (9, 10; 9A, 10A) comprend un émetteur de rayonnement optique (5) et un récepteur (6) de rayonnement optique placés respectivement dans ledit boîtier (2) et dans ladite corne (4), le trajet lumineux entre eux pouvant ainsi passer à travers une portion (P) du pavillon de l'oreille d'un porteur.

3. Equipement portable selon la revendication 2, **caractérisé en ce que** ledit émetteur de rayonnement optique (5) comprend une pluralité de sources lumineuses (12, 13) émettant sur des longueurs d'onde distinctes (λ1, λ2), situées de préférence dans le proche infrarouge.

4. Equipement portable selon la revendication 3, **caractérisé en ce que** ledit récepteur (6) comprend autant de groupes de détecteurs de rayonnement optique (14, 15) qu'il y a de sources de lumière dans l'émetteur (5) et **en ce que** ledit dispositif de mesure comprend également des moyens (16, 17) pour calculer la moyenne des signaux fournis par les détecteurs de chaque groupe.

5. Equipement portable selon l'une quelconque des revendications 1 à 4, **caractérisé en ce que** lesdits moyens de substitution/superposition de ladite unité de restitution sonore comprennent au moins un circuit de mixage (24, 26) connecté pour recevoir ledit signal représentatif de la fréquence cardiaque fourni par ledit dispositif de mesure (9, 10; 9A, 10A), ledit circuit de mixage (24, 26) étant connecté entre ledit transducteur sonore (3) et lesdits moyens fournissant ledit signal représentatif de la restitution sonore (22; 22A, 31).

6. Equipement portable selon la revendication 5, **caractérisé en ce que** ladite unité de restitution sonore comprend des moyens de synthèse vocale (23) connectés entre ledit dispositif de mesure (9, 10; 9A, 10A) et ledit circuit de mixage (24).

7. Equipement portable selon l'une quelconque des revendications 5 et 6, **caractérisé en ce que qu**'il comprend un comparateur (25) dont une première entrée reçoit le signal représentatif de la fréquence cardiaque et dont une seconde entrée est reliée à un générateur de seuil (27) dont le seuil est représentatif d'une valeur prédéterminée de la fréquence cardiaque, et dont la sortie est reliée audit circuit de mixage (26) pour envoyer dans ledit transducteur sonore (3) une information d'alarme, lorsque ladite valeur prédéterminée est dépassée par la valeur de la fréquence cardiaque fournie par ledit dispositif de mesure (9, 10, 9A, 10A).

8. Equipement suivant les revendications 6 et 7 prises ensemble, **caractérisé en ce qu**'un sélecteur (28) permet de choisir la reproduction dans ledit transducteur sonore (3) soit de ladite information d'alarme, soit dudit signal représentatif de la fréquence cardiaque fournie par ledit dispositif de mesure (9, 10).

9. Equipement selon l'une quelconque des revendications 1 à 8, **caractérisé en ce que** ladite unité de restitution sonore comprend un lecteur (B) d'un support d'information sonore tel qu'une cassette audio, un CD ou un DVD, connecté audit ensemble (2, 4) par un câble (C).

10. Equipement selon l'une quelconque des revendications 1 à 8, **caractérisé en ce que** ladite unité de restitution sonore (22A, 31) comprend un microphone (31) et **en ce qu'**il constitue une prothèse acoustique.

## Claims

1. Portable equipment including in combination:
- a measuring device (9, 10; 9A, 10A) for measuring the heart rate,
- a sound reproduction unit (B; 22A, 31) including means for supplying a signal representative of the sound reproduction (22; 22A, 31) for supplying sound information to a sound transducer (3),
- said measuring device (9, 10; 9A, 10A) and said sound transducer (3) are mounted at least in part in an assembly (2,4) adapted to be fixed to an ear of a wearer of the equipment, and
- said sound reproduction unit (B; 22A, 31) includes substitution/superimposition means (24, 26) for substituting for and/or superimposing on said signal representative of said sound reproduction a signal generated from signals from said measuring device (9, 10, 9A, 10A) and representative of said heart rate,
- said assembly (2, 4) takes the form of an ear cushion including a casing (2) accommodating said sound transducer (3) and adapted to be placed in front of the external auditory meatus opening of a wearer and a horn (4) attached to said casing (2) and adapted to be placed behind the external ear of a wearer,
**characterized in that**:
- said measuring device (9, 10, 9A, 10A) is accommodated in part in said casing (2) and in part in said horn (4),
- said assembly further includes an accelerometric device (8) delivering motion signals representative of the motion of a wearer when the assembly (2, 4) is fixed to an ear of said wearer and said accelerometric device (8) further including signal processing means (18, 20) using said motion signals for correcting artifacts due to motion in said signal representative of said heart rate.

2. The portable equipment claimed in claim 1, wherein said measuring device (9, 10, 9A, 10A) includes an optical radiation emitter (5) and an optical radiation receiver (6) respectively placed in said casing (2) and in said horn (4) so that the light path between them can pass through a portion (P) of the external ear of a wearer.

3. The portable equipment claimed in claim 2, wherein said optical radiation emitter (5) includes a plurality of light sources (12, 13) emitting at separate wavelengths (λ1, λ2) that are preferably near infra-red wavelengths.

4. The portable equipment claimed in claim 3, wherein said receiver (6) includes the same number of groups of optical radiation detectors (14, 15) as there are light sources in said emitter (5) and said measuring device also includes means (16, 17) for calculating the average of signals supplied by the detectors of each group.

5. The portable equipment claimed in any claim 1 to 4, wherein said substitution/superimposition means of said sound reproduction unit include at least a mixer circuit (24, 26) adapted to receive said signal representative of said heart rate supplied by said measuring device (9, 10; 9A, 10A) and connected between said sound transducer (3) and said means (22; 22A, 31) supplying said signal representative of the sound reproduction.

6. The portable equipment claimed in claim 5, wherein said sound reproduction unit includes voice synthesizer means (23) connected between said measuring device (9, 10, 9A, 10A) and said mixer circuit (24).

7. The portable equipment claimed in any claim 5 and 6 further including a comparator (25) having a first input adapted to receive said signal representative of said heart rate and a second input connected to a threshold generator (27) whose threshold is representative of a predetermined heart rate value and whose output is connected to said mixer circuit (26) to send alarm information to said sound transducer (3) if the heart rate supplied by said measuring device (9, 10; 9A, 10A) exceeds said predetermined heart rate value.

8. The portable equipment claimed in claims 6 and 7, wherein a selector (28) is adapted to select reproduction by said sound transducer (3) either of said alarm information or of said signal representative of said heart rate supplied by said measuring device (9, 10).

9. The portable equipment according to any claim 1 to 8, wherein said sound reproduction unit includes a reader (B) of a sound information medium, such as an audio cassette, a compact disc or a digital versatile disc, connected to said assembly (2,4) by a cable (C).

10. The portable equipment according to any claim 1 to 8, wherein said sound reproduction unit (22A, 31) includes a microphone (31) and wherein it constitutes a hearing aid.

## Patentansprüche

1. Tragbare Ausrüstung umfassend in Kombination:
- eine Herzfrequenzmessvorrichtung (9, 10; 9A, 10A),
- eine Audiowiedergabeeinheit (B; 22A, 31) umfassend Mittel, die ein repräsentatives Signal der Audiowiedergabe ausgeben (22; 22A, 31), um einem Schallwandler (3) akustische Information zu liefern,
- wobei die Messvorrichtung (9, 10; 9A, 10A) und der Schallwandler (3) mindestens teilweise in einer Einheit (2,4) eingebaut sind, die dafür ausgelegt ist, am Ohr eines Trägers der Ausrüstung befestigt zu werden und
- wobei die Audiowiedergabeeinheit (B; 22A, 31) Mittel (24, 26) umfasst, um das repräsentative Signal der Audiowiedergabe mit einem Signal zu ersetzen und/oder zu überlagern, das aus den Signalen der Messvorrichtung (9, 10, 9A, 10A) erzeugt wird und das repräsentativ für die Herzfrequenz ist,
- wobei die Einheit (2, 4), die dazu ausgelegt ist, am Ohr eines Trägers befestigt zu werden, die Form eines Kopfhörers aufweist, der ein Gehäuse (2), in dem der Wandler (3) eingebaut ist und das dazu bestimmt ist, vor der Öffnung des Gehörgangs eines Trägers angebracht zu werden und einen Bügel (4) aufweist, der mit dem Gehäuse (2) verbunden ist und dazu bestimmt ist, hinter der Ohrmuschel eines Trägers angebracht zu werden,
**dadurch gekennzeichnet, dass**:
- die Messvorrichtung (9, 10; 9A, 10A) teilweise im Gehäuse (2) und teilweise im Bügel (4) eingebaut ist,
- die Einheit außerdem eine akzelerometrische Vorrichtung (8) umfasst, die repräsentative Bewegungssignale der Trägerbewegung ausgibt, wenn die Einheit am Ohr des Trägers befestigt ist und die akzelerometrische Vorrichtung (8) außerdem Mittel (18, 20) zum Signalverarbeiten umfasst, die die Bewegungssignale nutzen, um Artefakte, die auf die Bewegung zurück zu führen sind, im repräsentativen Signal der Herzfrequenz zu korrigieren.

2. Tragbare Ausrüstung nach Anspruch 1, **dadurch gekennzeichnet, dass** die Messvorrichtung (9, 10; 9A, 10A) einen optischen Sender (5) und einen optischen Empfänger (6) umfasst, die jeweils im Gehäuse (2) und im Bügel (4) angebracht sind, wobei der Lichtweg zwischen ihnen so durch einen Abschnitt (P) der Ohrmuschel eines Trägers führen kann.

3. Tragbare Ausrüstung nach Anspruch 2, **dadurch gekennzeichnet, dass** der optische Sender (5) eine Vielzahl von Lichtquellen (12, 13) umfasst, die auf verschiedenen Wellenlängen (λ1 λ2) emittieren, die sich vorzugsweise im nahen Infrarotbereich befinden.

4. Tragbare Ausrüstung nach Anspruch 3, **dadurch gekennzeichnet, dass** der Empfänger (6) so viele Gruppen optischer Strahlungsdetektoren (14, 15) umfasst wie der Sender (5) Lichtquellen umfasst und dass die Messvorrichtung außerdem Mittel (16, 17) umfasst, um den Durchschnitt der Signale zu berechnen, die von den Detektoren jeder Gruppe ausgegeben werden.

5. Tragbare Ausrüstung nach einem der Ansprüche 1 bis 4, **dadurch gekennzeichnet, dass** die Mittel zum Ersetzen/Überlagern der Audiowiedergabeeinheit mindestens eine Schaltung zum Mischen (24, 26) umfassen, die angeschlossen ist, um das repräsentative Signal der Herzfrequenz zu empfangen, das von der Messvorrichtung (9, 10; 9A, 10A) ausgegeben wird, wobei die Schaltung zum Mischen (24, 26) zwischen dem Schallwandler (3) und den Mitteln, die das repräsentative Signal der Audiowiedergabe (22; 22A, 31) ausgeben, angeschlossen ist.

6. Tragbare Ausrüstung nach Anspruch 5, **dadurch gekennzeichnet, dass** die Audiowiedergabeeinheit Mittel zur Sprachsynthese (23) umfasst, die zwischen der Messvorrichtung (9, 10; 9A, 10A) und der Schaltung zum Mischen (24) angeschlossen sind.

7. Tragbare Ausrüstung nach einem der Ansprüche 5 und 6 **dadurch gekennzeichnet, dass** sie einen Komparator (25) umfasst, von dem ein erster Eingang das repräsentative Signal der Herzfrequenz empfängt und ein zweiter Eingang mit einem Schwellenwertgenerator (27) verbunden ist, dessen Schwelle für einen vordefinierten Wert der Herzfrequenz repräsentativ ist und von dem der Ausgang mit der Schaltung zum Mischen (26) verbunden ist, um in den Schallwandler (3) eine Alarminformation zu senden, wenn der vordefinierte Wert durch den Herzfrequenzwert überschritten wird, der von der Messvorrichtung (9, 10, 9A, 10A) ausgegeben wird.

8. Ausrüstung nach Ansprüchen 6 und 7 zusammen genommen, **dadurch gekennzeichnet, dass** ein Wählschalter (28) es ermöglicht, die Wiedergabe in dem Schallwandler (3) entweder der Alarminformation oder des repräsentativen Signals der Herzfrequenz, das von der Messvorrichtung (9, 10) ausgegeben wird, auszuwählen.

9. Ausrüstung nach einem der Ansprüche 1 bis 8, **dadurch gekennzeichnet, dass** die Audiowiedergabeeinheit ein Abspielgerät (B) für einen akustischen Informationsträger wie eine Audiokassette, eine CD oder eine DVD umfasst, das mit der Einheit (2, 4) durch ein Kabel (C) verbunden ist.

10. Ausrüstung nach einem der Ansprüche 1 bis 8, **dadurch gekennzeichnet, dass** die Audiowiedergabeeinheit (22A, 31) ein Mikrophon (31) umfasst und dass sie eine akustische Prothese bildet.
